(19) Europäisches Patentamt
European Patent Office
Office européen des brevets

(11) Veröffentlichungsnummer: **0 190 606**
**B1**

(12) # EUROPÄISCHE PATENTSCHRIFT

(45) Veröffentlichungstag der Patentschrift: **09.05.90**

(51) Int. Cl.[5]: **B01J 20/32**, C07K 3/20, C07K 15/26

(21) Anmeldenummer: **86100787.0**

(22) Anmeldetag: **22.01.86**

(54) **Adsorbens für die Reinigung von Proteinen.**

(30) Priorität: **04.02.85 CH 490/85**

(43) Veröffentlichungstag der Anmeldung: **13.08.86 Patentblatt 86/33**

(45) Bekanntmachung des Hinweises auf die Patenterteilung: **09.05.90 Patentblatt 90/19**

(84) Benannte Vertragsstaaten: **AT BE CH DE FR GB IT LI LU NL SE**

(56) Entgegenhaltungen:
**EP-A- 0 064 833**
**EP-A- 0 122 969**
**WO-A-79/00541**
**GB-A- 1 602 432**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE AG, Postfach 3255, CH-4002 Basel(CH)**

(72) Erfinder: **Eggimann, Bernhard, Dr., Eulerstrasse 83, CH-4051 Basel(CH)**
Erfinder: **Hochuli, Erich, Dr., Kirchackerstrasse 25, CH-4411 Arisdorf(CH)**
Erfinder: **Schacher, Alfred, Im Niederholzboden 58, CH-4125 Riehen(CH)**

(74) Vertreter: **Lederer, Franz, Dr. et al, Van der Werth, Lederer & Riederer Patentanwälte Lucile-Grahn-Strasse 22, D-8000 München 80(DE)**

**EP 0 190 606 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Harze, bzw. Adsorbentien, die für die Affinitäts-Chromatographie geeignet sind und daher für die Reinigung von Proteinen, insbesondere von Interferonen, verwendet werden können, sowie ein Verfahren zur Reinigung von Proteinen, insbesondere Interferonen, mittels dieser neuen Harze.

Die erfindungsgemässen Harze bestehen aus einer elektroneutralen Trägermatrix, $-O-CH_2-CH(OH)-CH_2-NH$-Gruppen als Spacer und einem an die Spacer-Aminogruppe gebundenen Triazinfarbstoff als reaktivem Liganden.

Als elektroneutrale Trägermatrix kommen Materialien in Frage, die in der Affinitäts- und Gelchromatographie verwendet werden, wie vernetzte Dextrane, Agarose (insbesondere in der unter dem Warenzeichen Sepharose® bekannten Form) oder Polyacrylamide.

Als Triazinfarbstoffe kommen die aus der Affinitäts-Chromatographie bereits bekannten Reaktivfarbstoffe, wie die zur Reaktivblau- und Reaktivrot-Gruppe gehörenden, in Frage, zu deren bekanntesten Vertretern Cibacron®-Blau, Cibacron®-Brillant Rot und Procion®-Rot zählen (siehe z.B. Int. J Biochem. 13, 33-40 [1981] und J. Chromatog. 165, 301-319 [1979]) und die im Handel erhältlich sind.

In der Affinitäts-Chromatographie zur Reinigung von Proteinen, beispielsweise von Interferonen, werden bereits Adsorbentien eingesetzt, die aus einer Trägermatrix, beispielsweise Agarose, vernetzten Dextranen oder Polyacrylamiden, bestehen, an die als reaktive Gruppen Farbstoffe, insbesondere Triazinfarbstoffe wie die vorstehend genannten (J. Chromatog. 165, 301 [1979], Dean et al.), oder Antikörper, die für die zu reinigenden Proteine spezifisch sind (polyklonale oder monoklonale) direkt kovalent gebunden sind. Derartige Adsorbentien, die sich z.B. für die Reinigung von Interferon eignen, sind beispielsweise in den US-Patenten 4.172.071 (De Maeyer et al.), 4.278,661 (Knight, Jr.) und 4.289.689 (Friesen et al.), sowie in J. Biol. Chem. 256, 9750 [1981] (Staehelin et al.) und Scand. J. Immunol. 8, 429 [1978] (Berg et al.) beschrieben.

Die erfindungsgemässen Adsorbentien zeichnen sich gegenüber den bekannten vor allem dadurch aus. dass sie mechanisch stärker belastbar sind und höhere Durchflussraten als die vergleichbaren bekannten Harze, wie z.B. Blau-Sepharose®, erlauben, was sie besonders für Arbeiten im grosstechnischen Massstab geeignet macht. Dieser Vorteil ist besonders entscheidend wenn für die Chromatographie Puffer mit hoher Salzkonzentration oder Puffergemische aus organischen Lösungsmitteln und wässrigen Salzlösungen von hoher Viskosität verwendet werden.

Es hat sich gezeigt, dass sich die erfindungsgemässen Harze durch eine besonders hohe Spezifität gegenüber β-Interferon (Fibroblasten-Interferon) auszeichnen und daher besonders geeignet sind für die Reinigung von β-Interferon und dessen Analogen, insbesondere von rekombinantem β-Interferon. Sie eignen sich aber darüberhinaus auf Grund ihrer vorteilhaften Eigenschaften nicht nur für die Reinigung anderer Interferone (α-, γ-, hybride Interferone) bzw. Lymphokine, wie Interleukin-2, unterschiedlicher Provenienz sondern von Proteinen ganz allgemein, wobei je nach Produkt die Auswahl der am besten geeigneten Trägermatrix sowie des reaktiven Liganden und die Optimierung der Verfahrensbedingungen im Rahmen des allgemeinen Wissens des Fachmanns erfolgen kann. Die Harze können selbstverständlich batch-weise oder in kontinuierlich zu betreibenden Säulen verwendet werden.

Die Herstellung der erfindungsgemässen Adsorbentien kann in an sich bekannter Weise erfolgen, wobei zunächst die Matrix funktionalisiert wird (Einführung des Spacers) und dann über den Spacer der gewünschte Farbstoff kovalent gebunden wird. Bei Agarose als Ausgangsmaterial setzt man beispielsweise mit Epibromhydrin in alkalischem Medium um, öffnet den Epoxidring durch Umsetzung mit wässrigem Ammoniak, so dass man 3-Amino-2-hydroxypropyl-agarose erhält (vgl. J. Solid-Phase Biochem. 1, 33 [1976]). Nach der Umsetzung mit dem gewünschten Farbstoff können überschüssige freie Spacer-3-Aminofunktionen beispielsweise durch Umsetzung mit Kaliumcyanat (Ueberführung in Ureidogruppen) blockiert werden. In analoger Weise können Polyacrylharze, die freie Hydroxylgruppe enthalten, in erfindungsgemässe Adsorbentien überführt werden.

Ausgangsmaterial für einen bevorzugten erfindungsgemässen Harz-Typ auf der Basis einer Polyacrylamid-Trägermatrix ist ein Oxiran-Polyacrylharz, das

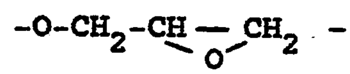

-Gruppen enthält und das unter dem Markennamen Eupergit®-C der Firma Röhm Pharma GmbH, Darmstadt, im Handel ist. Die Epoxypropylgruppen des Eupergit®-C können durch Behandlung mit wässrigem Ammoniak in 3-Amino-2-hydroxypropylgruppen übergeführt werden. So erhaltenes Aminoeupergit kann dann in an sich bekannter Weise durch Umsetzung mit einem Reaktivfarbstoff, wie vorstehend angegeben, in das gewünschte erfindungsgemässe Harz übergeführt werden.

Besonders bevorzugte Reaktivfarbstoffe im Zusammenhang mit der vorliegenden Erfindung sind Cibacron®-Blau 3G-A bzw. F3G-A und Cibacron®-Brillant Rot 4G-E.

Die folgenden Beispiele illustrieren die Herstellung einiger erfindungsgemässer Adsorbentien sowie deren Verwendung bei der Reinigung von rekombinantem Human-Fibroblasten-Interferon (rIFN-β), wobei die Mengenverhältnisse der Reaktionspartner nicht kritisch sind und je nach Problemstellung, d.h. je nach dem zu reinigenden Protein bzw. Proteingemisch, variiert werden können. Auf diese Weise lassen sich erfindungsgemässe Adsorbentien erhalten, die sich voneinander in Spacer- bzw. Farbstoffgehalt unterscheiden. Die optimalen Verhältnisse lassen sich vom Fachmann leicht empirisch ermitteln. Für die Reinigung von Fibroblasten-Interferon (IFN-β) hat sich beispielsweise ein Verhältnis von etwa 7-8 µMol Cibacron®-Blau pro ml Gel als sehr günstig erwiesen, jedoch können von etwa 2 bis zu etwa 15 µMol Farbstoff eingesetzt werden.

Die Reinigung der Proteine mit den erfindungsgemässen Harzen kann analog zu der von Friesen et al. (Arch. Biochem. Biophys. 206, 432-450 [1981] beschriebenen Chromatographie von natürlichem Fibroblasten-Interferon aus menschlichem Gewebe an Blau-Dextran Sepharose® durchgeführt werden.

Der als Ausgangsmaterial verwendete rIFN-β-Rohextrakt wurde nach der Methode von Goeddel et al. (Nucleic Acids Res. 8, 4057-4074 [1980] hergestellt.

Die Elution wurde mit einem Photometer mit Durchflusszelle (Wellenlänge280 nm) und Schreiber verfolgt. Die Eluate wurden mit Hilfe eines Fraktionensammlers aufgefangen und fraktioniert.

Die erhaltene reine Interferon-Lösung kann zur Entfernung des Ethylenglykols gegen 0,05 M Natriumacetatpuffer (pH 5.0) dialysiert und mittels Ultrafiltration aufkonzentriert werden.

Die Bestimmung des Proteingehaltes erfolgte nach der Methode von Lowry et al. (J. Biol. Chem. 193 , 265-275 [1951] unter Verwendung von Serumalbumin als Standard.

Die Reinheitsbestimmung erfolgte mittels SDS-PAGE, wie von Laemmli et al. beschrieben (Nature 277, 680-685 [1970]), mit der Modifikation, dass die Elektrophorese unter nicht-reduzierenden Bedingungen (d.h. ohne Zusatz von 2-Mercaptoethanol) durchgeführt wurde. Als Proteinstandard-Marker wurde ein Gemisch von Lysozym (MG: 14'400), Trypsin-Inhibitor (MG: 21'500), Carbonil-Anhydrase (MG: 31'000), Ovalbumin (MG: 45'000), Rinderserum-Albumin (MG: 66'200) und Phosphorylase B (MG: 92'500) verwendet.

Beispiel 1

Herstellung eines Agarose-Cibacron® Blau-Harzes

50 ml Agarosegel (Sepharose® CL-6B) wurden auf einer Glasfilternutsche zweimal mit ca. 250 ml Wasser gewaschen, in einen 200 ml-Sulfierkolben überführt und mit Wasser auf ein Volumen von 100 ml gebracht. Nach Zusatz von 8 ml 4N NaOH und 2 ml Epibromhydrin wurde das Gemisch 5 Stunden bei 30°C gerührt. Das aktivierte Harz wurde auf der Nutsche mit Wasser neutral gewaschen und zurück in den Sulfierkolben transferiert. Nach Zugabe von 100 ml 5% Ammoniaklösung (Gew./Vol.-%) wurde das Gemisch über Nacht bei Raumtemperatur gerührt. Dann wurde das Harz erneut neutral gewaschen und mit Wasser auf ein Volumen von 100 ml gebracht. Nach Zugabe von 1 g $Na_2CO_3$ und 271 mg Cibacron® Blau 3G-A (7 µMol/ml Gel) rührte man während 20 Stunden bei 50°C. Auf der Glasfilternutsche wurde das fertige Gel nacheinander mit je ca. 500 ml Wasser. Methanol/Wasser (1:1) und Wasser farblos gewaschen.

Beispiel 2

Herstellung eines Aminoeupergit-Cibacron®Blau-Harzes

250 ml 5% Ammoniaklösung (Gew./Vol.-%) wurden in einem 500 ml-Rundkolben vorgelegt. Nachdem man 25 g Eupergit®C portionenweise zugegeben hatte. wurde 6 Stunden bei Raumtemperatur mit einem Flügelrührer langsam gerührt Dann wurde die Rührung abgestellt und nach drei Minuten der Ueberstand abgesaugt. Mit 100 ml Wasser wurde das Harz wieder aufgeschlämmt und nach 3 Minuten der Ueberstand wieder abgesaugt. Diesen Vorgang wiederholte man viermal. Das so erhaltene feuchte Aminoeupergit wurde in einen 500 ml 4-Halskolben überführt, mit 200 ml 0,2M Dinatriumhydrogenphosphat und 688 mg Cibacron®-Blau 3G-A (8 µMol pro ml Gel) versetzt und 20 Stunden bei 40°C langsam gerührt. Dann wurde das Harz auf einer Glasfilternutsche nacheinander mit je 500 ml Wasser. 0,01N Salzsäure und Wasser gewaschen. Anschliessend wurde das Gel ins Reaktionsgefäss zurüchtransferiert, mit 220 ml Wasser und 2,08 g Kaliumcyanat versetzt und während 15 Stunden bei Raumtemperatur langsam gerührt. Das fertige Harz (ca. 100 ml) wurde schliesslich auf der Glasfilternutsche nacheinander mit je 1 l Wasser, Methanol/Wasser (1:1) und Wasser gewaschen.

Beispiel 3

Herstellung eines Aminoeupergit-Cibacron® Brilliant Rot-Harzes

100 ml Aminoeupergit, aus 25 g Eupergit®C nach der in Beispiel 2 beschriebenen Methode hergestellt, wurden in einem 500 ml Kolben mit 200 ml 0,2M Dinatriumhydrogenphosphat und. 1.029 g Cibacron® Brillant Rot 4G-E (7 µMol Farbstoff pro ml Gel) versetzt. Das Reaktionsgemisch wurde während 15 Stunden bei 40°C langsam gerührt. Anschliessend wurde das Harz auf der Nutsche nacheinander mit je 500 ml Wasser, 1M Kaliumchloridlösung, 0,01N Salzsäure und wieder mit Wasser gewaschen.

Dann wurde das Harz ins Reaktionsgefäss zurückgebracht und während ca. 15 Stunden in 200 ml Wasser mit 2,08 g Kaliumcyanat gerührt. Das gebrauchsfertige Harz (ca. 100 ml) wurde in zu Beispiel 2 analoger Weise mit Wasser, Methanol/Wasser und wieder mit Wasser gewaschen.

Beispiel 4

Reinigung von rIFN-β an einem Agarose-Cibacron® Blau-Harz

Eine Säule (2.6 x 4.7 cm). enthaltend 25 ml des gemäss Beispiel 1 hergestellten Agarose-Cibacron® Blau-Harzes, wurde mit ca. 200 ml 0.05M Phosphatpuffer (pH 7,2, 1M NaCl) äquilibriert. Die Säule wurde bei 4°C mit 1050 ml rIFN-β-Rohextrakt, erhalten aus 28 g tiefgefrorenen E. coli-Zellen gemäss Goeddel et al. (Nucleic Acids Res.8, 4057 [1980]),

mit einer Flussrate von 8 cm/Stunde (ml/cm²/Std.) beladen. Anschliessend wurde bei Raumtemperatur nacheinander mit jeweils 100 ml Phosphatpuffer (0.05M, pH 7,2, 1M NaCl), enthaltend 15 bzw. 30 Vol./Vol.-% Ethylenglykol gewaschen und mit Phosphatpuffer (0.05M, pH 7,2, 1M NaCl), enthaltend 50 Vol./Vol.-% Ethylenglykol, das Interferon eluiert. Es wurden 2,2 mg reines rIFN-β (Reinheit >95% gemäss SDS-PAGE) in 66 ml Elutionspuffer erhalten.

Anschliessend wurde die Säule durch Waschen mit Phosphatpuffer (0.05M, pH 7,2, 1M NaCl), enthaltend 70 Vol./Vol.-% Ethylenglykol, wieder einsatzbereit gemacht.

Beispiel 5

Reinigung von rIFN-β an einem Aminoeupergit-Cibacron® Blau-Harz

a) Eine Säule (2,6 x 7 cm), enthaltend 37 ml des gemäss Beispiel 2 hergestellten Aminoeupergit-Cibacron®Blau-Harzes, wurde mit ca. 200 ml 0.05M Phosphatpuffer (pH 7,2, 1M NaCl) äquilibriert. Die Säule wurde dann bei 6°C mit 700 ml rIFN-β-Rohextrakt, erhalten aus 14,8 g tiefgefrorenen E. coli-Zellen gemäss Goeddel et al. (Nucleic Acids Res.8, 4057 [1980]), bei einer Flussrate von 38 cm/Stunde beladen. Anschliessend wurde die Säule bei Raumtemperatur nacheinander mit 130 ml Phosphatpuffer (0.05M. pH 7,2, 1M NaCl), enthaltend 15 bzw. 30 Vol./Vol.-% Ethylenglykol, sowie 60 ml Phosphatpuffer (0.05M, pH 7,2, 1M NaCl), enthaltend 30 Vol./Vol.-% Ethylenglykol, gewaschen und das Interferon mit Phosphatpuffer (0.05M, pH 7,2, 1M NaCl), enthaltend 50 Vol./Vol.-% Ethylenglykol, eluiert. Es wurden 1.15 mg reines rIFN-β (Reinheit >95% gemäss SDS-PAGE) in 38 ml Elutionspuffer erhalten.

Anschliessend wurde die Säule durch Waschen mit Phosphatpuffer (0.05M, pH 7,2, 1M NaCl), enthaltend 70 Vol./Vol.-% Ethylenglykol, wieder einsatzbereit gemacht.

b) In zu a) analoger Weise wurde eine Säule (14 x 13 cm), enthaltend 2 l des gemäss Beispiel 2 hergestellten Harzes, mit 60 l rIFN-β-Rohextrakt, erhalten aus 2 kg E. coli-Zellen (gemäss Goeddel et al.), bei einer Flussrate von 39 cm/Stunde beladen, gewaschen und eluiert, wobei 300 mg reines rIFN-β (Reinheit >95% gemäss SDS-PAGE) in 2,3 l Elutionspuffer erhalten wurden.

## Patentansprüche

1. Adsorbens, dadurch gekennzeichnet, dass es aus einer elektroneutralen Trägermatrix, $-O-CH_2-CH(OH)-CH_2-NH$-Gruppen als Spacer und einem an die Spacer-Aminogruppe gebundenen Triazinfarbstoff besteht.

2. Adsorbens gemäss Anspruch 1, dadurch gekennzeichnet, dass die Trägermatrix Agarose ist.

3. Adsorbens gemäss Anspruch 1, dadurch gekennzeichnet, dass die Trägermatrix ein Polyacrylamidharz ist.

4. Adsorbens gemäss einem der Ansprüche 1-3, dadurch gekennzeichnet, dass der Triazinfarbstoff ein Reaktivfarbstoff vom Typ der Cibacron®-Gruppe ist.

5. Adsorbens gemäss einem der Ansprüche 1, 2 oder 4, dadurch gekennzeichnet, dass Cibacron® Blau über $-O-CH_2-CH(OH)-CH_2-NH$-Gruppen an Agarose gebunden ist.

6. Adsorbens gemäss einem der Ansprüche 1, 3 oder 4, dadurch gekennzeichnet, dass Cibacron® Blau an Aminoeupergit gebunden ist.

7. Adsorbens gemäss einem der Ansprüche 1, 3 oder 4, dadurch gekennzeichnet, dass Cibacron® Brillant Rot an Aminoeupergit® gebunden ist.

8. Verfahren zur Herstellung eines Adsorbens für die Affinitäts-Chromatographie, dadurch gekennzeichnet, dass man eine mit 3-Amino-2-hydroxypropylgruppen funktionalisierte Matrix mit einem Triazinfarbstoff umsetzt und gegebenenfalls überschüssige freie 3-Aminofunktionen blockiert.

9. Verfahren gemäss Anspruch 8, dadurch gekennzeichnet, dass man Aminoeupergit mit Cibacron® Blau oder Cibacron® Brillant Rot umsetzt.

10. Verfahren zur Reinigung von Proteinen, dadurch gekennzeichnet, dass man sie der Affinitäts-Chromatographie an einem Adsorbens gemäss den Ansprüchen 1-7 unterwirft.

11. Verfahren gemäss Anspruch 10, dadurch gekennzeichnet, dass man von einer Interferon-Lösung ausgeht.

12. Verfahren gemäss Anspruch 10 oder 11, dadurch gekennzeichnet, dass man von einer Lösung rekombinanten Fibroblasten-Interferons ausgeht.

13. Verwendung eines Adsorbens gemäss einem der Ansprüche 1-7 in der Affinitäts-Chromatographie.

14. Verwendung eines Adsorbens gemäss einem der Ansprüche 1-7 zur Reinigung von Proteinen.

15. Verwendung eines Adsorbens gemäss einem der Ansprüche 5-7 zur Reinigung von IFN-β.

## Claim

1. An adsorbent, characterized in that it consists of an electroneutral carrier matrix, $-O-CH_2-CH(OH)-CH_2-NH-$ groups as spacer and a triazine colouring substance bonded to the spacer amino group.

2. An adsorbent according to claim 1, characterized in that the carrier matrix is agarose.

3. An adsorbent according to claim 1, characterized in that the carrier matrix is a polyacrylamide resin.

4. An adsorbent according to any one of claims 1–3, characterized in that the triazine colouring substance is a reactive colouring substance of the Cibacron® group.

5. An adsorbent according to any one of claims 1, 2 or 4, characterized in that Cibacron® Blue is bonded to agarose via $-O-CH_2-CH(OH)-CH_2-NH-$ groups.

6. An adsorbent according to any one of claims 1, 3 or 4, characterized in that Cibacron® Blue is bonded to Aminoeupergit®.

7. An adsorbent according to any one of claims 1,

3 or 4, characterized in that Cibacron® Brilliant Red is bonded to Aminoeupergit®.

8. A process for the manufacture of an adsorbent for affinity chromatography, characterized in that one reacts a matrix functionalized with 3-amino-2-hydroxypropyl groups with a triazine colouring substance and, if necessary, blocking excess free 3-amino functions.

9. A process according to claim 8, characterized in that one reacts Aminoeupergit® with Cibacron® Blue or Cibacron® Brilliant Red.

10. A process for the purification of proteins, characterized in that one subjects them to affinity chromatography on an adsorbent according to claims 1–7.

11. A process according to claim 10, characterized in that one starts with an interferon solution.

12. A process according to claim 10 or 11, characterized in that one starts with a solution of recombinant fibroblast interferon.

13. The use of an adsorbent according to any one of claims 1–7 in affinity chromatography.

14. The use of an adsorbent according to any one of claims 1–7 for the purification of proteins.

15. The use of an adsorbent according to any one of claims 5–7 of the purification of IFN-β.

**Revendications**

1. Adsorbant caractérisé en ce qu'il consiste en une gangue de support électriquement neutre, des groupes $-O-CH_2-CH-(OH)-CH_2-NH-$ en tant que groupes de liaison, et un colorant triazinique fixé sur le groupe amino du groupe de liaison.

2. Adsorbant selon la revendication 1, caractérisé en ce que la gangue consiste en agarose.

3. Adsorbant selon la revendication 1, caractérisé en ce que la gangue de support consiste en une résine de polyacrylamide.

4. Adsorbant selon l'une des revendications 1 à 3, caractérisé en ce que le colorant triazinique est un colorant réactif du type du commerce Cibacron®.

5. Adsorbant selon l'une des revendications 1, 2 ou 4, caractérisé en ce que le Bleu Cibacron® est fixé sur l'agarose par l'intermédiaire des groupes $-O-CH_2-CH-(OH)-CH_2-NH-$.

6. Adsorbant selon l'une des revendications 1, 3 ou 4, caractérisé en ce que le Bleu Cibacron® est fixé sur le produit du commerce Aminoeupergit®.

7. Adsorbant selon l'une des revendications 1, 3 ou 4, caractérisé en ce que le Rouge Brilliant Cibacron® est fixé sur le produit du commerce Aminoeupergit®.

8. Procédé de préparation d'un adsorbant pour la chromatographie d'affinité, caractérisé en ce que l'on fait réagir une gangue fonctionnalisée par des groupes 3-amino-2-hydroxypropyle avec un colorant triazinique et le cas échéant on bloque l'excès de fonctions 3-amino libres.

9. Procédé selon la revendication 8, caractérisé en ce que l'on fait réagir l'Aminoeupergit® avec le Bleu Cibacron® ou le Rouge Brilliant Cibacron®.

10. Procédé pour purifier des protéines, caractérisé en ce qu'on les soumet à la chromatographie d'affinité sur un adsorbant selon les revendications 1 à 7.

11. Procédé selon la revendication 10, caractérisé en ce que l'on part d'une solution d'interféron.

12. Procédé selon la revendication 10 ou 11, caractérisé en ce que l'on part d'une solution d'interféron recombinant de fibroblastes.

13. Utilisation d'un adsorbant selon l'une des revendications 1 à 7 dans la chromatographie d'affinité.

14. Utilisation d'un adsorbant selon l'une des revendications 1 à 7 pour la purification de protéines.

15. Utilisation d'un adsorbant selon l'une des revendications 5 à 7 pour la purification de l'IFN-bêta.